Europäisches Patentamt

European Patent Office

Office européen des brevets

(19)

(11) Veröffentlichungsnummer: **0 083 700**
**B1**

(12) # EUROPÄISCHE PATENTSCHRIFT

(45) Veröffentlichungstag der Patentschrift:
**27.12.84**

(21) Anmeldenummer: **82110059.1**

(22) Anmeldetag: **30.10.82**

(51) Int. Cl.³: **C 07 C 37/48**, C 07 C 37/14,
C 07 C 39/06

(54) **Verfahren zur Herstellung von p-tert.-Octylphenol durch katalytische Alkylierung von Phenol.**

(30) Priorität: **29.12.81 DE 3151693**

(43) Veröffentlichungstag der Anmeldung:
**20.07.83 Patentblatt 83/29**

(45) Bekanntmachung des Hinweises auf die Patenterteilung:
**27.12.84 Patentblatt 84/52**

(84) Benannte Vertragsstaaten:
**BE CH DE FR GB IT LI NL**

(56) Entgegenhaltungen:
**FR - A - 1 264 120**
**FR - A - 1 301 483**
**FR - A - 1 336 081**
**FR - A - 2 228 749**
**US - A - 3 422 157**

**JOURNAL OF ORGANIC CHEMISTRY, Band 22, Nr. 8, 14. August 1957, Seiten 988-989, Easton, USA B. LOEV et al.: "Cation Exchange Resins as Catalysts in the Alkylation of Phenols"**
**CHEMICAL ABSTRACTS, Band 95, Nr. 10, November 1981, Seite 693, Nr. 168698u, Columbus, Ohio, USA V.D. SUKHOVERKHOV et al.: "Alkylation of phenol and disproportionation of alkylphenols in the presence of cation exchangers"**

(73) Patentinhaber: **CHEMISCHE WERKE HÜLS AG, - RSP Patente / PB 15 - Postfach 13 20, D-4370 Marl 1 (DE)**

(72) Erfinder: **Alfs, Helmut, Bitterfelder Strasse 47, D-4370 Marl (DE)**
Erfinder: **Böxkes, Werner, Dr., Oppauer Strasse 2, D-4370 Marl (DE)**
Erfinder: **Vangermain, Erwin, Dr., Leverkusener Strasse 1, D-4370 Marl (DE)**

## Beschreibung

Alkylphenole stellt man technisch vorwiegend durch katalytische Alkylierung von Phenol mit Olefinen her. Die Katalyse bei diesen Friedel-Crafts-Reaktionen erfolgt entweder homogen (Schwefelsäure, Phosphorsäure, Bortrigluorid, Aluminiumchlorid usw.) oder heterogen (stark saure Kationentauscher, aktivierte Tonerden). Da die homogene Katalyse Probleme hinsichtlich des Umweltschutzes und der Korrosion zur Folge hat, wird die heterogene Katalyse mit sauren Ionenaustauschern, insbesondere mit Harzen vom Typ des sulfonierten, mit Divinylbenzol vernetzten Polystyrols, bevorzugt. Man verwendet hierbei Harze, deren Kornverteilungsmaximum bei 0,3 bis 1,3 mm liegt, und ordnet sie in Festbettreaktoren an, die je nach Strömungsgeschwindigkeit des Reaktionsgemisches von unten nach oben oder von oben nach unten durchströmt werden. Die Reaktivität bzw. Reaktionsgeschwindigkeit hängt hierbei u.a. von den eingesetzten Reaktionspartnern, aber auch von den Eigenschaften der Ionentauscher (Vernetzungsgrad, Sulfonierungsgrad, Austauschkapazität u.a.) ab. Insgesamt ist aber trotz grosstechnischen Einsatzes von Ionenaustauschern zur Katalyse die Wirkungsweise in Abhängigkeit von den Einsatzstoffen, insbesondere bei Alkylierungen weitestgehend unerforscht.

Ein verfahrenskritischer Schritt bei der technischen Durchführung dieser Reaktionen ist die Abführung der Reaktionswärme. Nach der US-PS Nr. 3422157 löst man dieses Problem, indem man grosse Mengen des Reaktionsgemisches aus einem ersten Reaktor über einen Wärmeaustauscher in diesen zurückführt und vergleichsweise geringe Mengen in einen zweiten Reaktor. Man kann aber auch, wie in der FR-PS Nr. 2228749 beschrieben, durch im Reaktor liegende Schlangenkühlung die Temperatur steuern. Nach der DE-PS Nr. 2346273 (= US-PS Nr. 4168390 = GB-PS Nr. 1481568) gelingt es, den Wärmestau zu verhindern, indem man das Aktivitätsniveau durch zwei hintereinander geschaltete Reaktoren mit unterschiedlichen Katalysatoraktivitäten beeinflusst. Dies erreicht man, indem man beispielsweise die Wasserstoffionen der Sulfogruppe teilweise durch Aluminiumionen ersetzt. Zusätzlich kann aber auch das Selektivitätsverhalten dieser Katalysatoren durch Austausch von H-Ionen mit Metallionen modifiziert werden. Häufig ist damit eine gleichzeitige Verringerung der Reaktionsgeschwindigkeit verbunden (H. Widdecke, „Ionenaustauscher als polymere Katalysatoren zur Alkylierung von Aromaten", Dissertation 1978, TU-Braunschweig, S. 131).

Besonders bemerkenswert ist die bekannte Abhängigkeit des heterogen katalysierten Alkylierungsvorganges von Wasser, das sowohl die Reaktionsgeschwindigkeit als auch die Selektivität beeinflusst. So wurde gefunden, dass bei der Umsetzung von Phenol mit Propen bei einer Reaktionstemperatur von 75°C und einem Zusatz von 10 Gew.-% Wasser der Phenolethergehalt um den Faktor 6,3 ansteigt und der Umsatz bei gleicher Reaktionszeit um den Faktor 3,3 fällt. Das für Wirtschaftlichkeitsüberlegungen wichtige Verhältnis von Mono-/Dialkylphenol wurde hierbei dagegen nicht verändert (H. Widdecke, S. 125). Erhöht man bei sonst gleichen Bedingungen die Temperatur auf 125°C, dann wird die Etherbildung stark zurückgedrängt und die Umsatzgeschwindigkeit um den Faktor 4,4 erhöht. Im „Journal of Org. Chemistry", 22, 8 (1957), S. 988, Spalte 2, Zeilen 10 bis 15, wird dagegen darauf hingewiesen, dass Wasser dem Reaktionsgemisch ferngehalten werden muss. Zudem sind die aus den niedrigen Temperaturen von 70 bis 75°C resultierenden langen Verweilzeiten von 24 h technisch uninteressant (S. 988, Spalte 2, Zeilen 48 bis S. 989, Spalte 1, Zeile 6).

Undurchsichtig sind auch die Alkylierungsreaktionen mit Isobuten und dessen Oligomeren. So wird beispielsweise im Gegensatz zur Alkylierung von Benzol mit Isobuten bei der Phenolalkylierung überraschend keine Oligomerisierung beobachtet (H. Widdecke, S. 118). Bei letzter Reaktion findet nämlich ausschliesslich eine Alkylierung des Phenols statt. Die Reaktion unterscheidet sich aber von anderen Alkylierungsreaktionen noch dadurch, dass eine Umalkylierung vom o-tert.-Butylphenol zum p-Isomeren möglich ist. Hierbei geht man davon aus, dass sowohl Isomerisierungs- als auch Transalkylierungsmechanismen ablaufen.

Die Kenntnis dieser Reaktionsverläufe konnte aber nicht annähernd die experimentellen Ergebnisse bei der Alkylierung von Phenol mit Diisobuten erklären. Bei dieser Reaktion wird nämlich gefunden, dass nicht, wie eigentlich erwartet, vorwiegend p-tert.-Octylphenol gebildet wird, sondern fast quantitativ p-tert.-Butylphenol. Über den Ablauf dieser Reaktion können wieder nur Vermutungen angestellt werden, die entweder eine Spaltung des Diisobutenmoleküls als ersten Schritt oder aber eine Disproportionierung des zunächst gebildeten Octylphenols möglich erscheinen lassen. Die Butylphenolbildung lässt sich durch Zusatz von 1 bis 2% Wasser, bezogen auf eingesetztes Phenol, bei Reaktionstemperaturen von 85 bis 110, insbesondere von 100 bis 105°C, unterdrükken (FR-PS Nr. 2228749), wobei vorher der Katalysator noch mit 10 bis 15% seines Gewichtes mit Wasser getränkt wurde. Bei diesen Reaktionsbedingungen ist aber die Reaktionsgeschwindigkeit aufgrund der hemmenden Wirkung des Wassers erwartungsgemäss sehr gering und der Anteil an unerwünschten Dialkylphenolen relativ hoch. Bei Temperaturen zwischen 120 und 130°C und ohne Wasserzusatz erhält man bei dem Verfahren der FR-PS Nr. 2228749 nur noch sehr geringe Mengen an Octylphenol, jedoch in hoher Ausbeute (94 bis 96%) p-tert.-Butylphenol.

Daraus ergab sich die Aufgabe, ein Verfahren zu finden, das die unwirtschaftlich langen Verweilzeiten verkürzt und die Bildung von unerwünschten Nebenprodukten, wie p-tert.-Butylphenol und Dialkylphenole, vermeidet.

Diese Aufgabe wird erfindungsgemäss entsprechend den Angaben der Patentansprüche gelöst.

Überraschenderweise erhält man bei dem erfindungsgemässen Verfahren bei kurzen Verweilzeiten und hohen Raum-Zeit-Ausbeuten p-tert.-Octylphenol in hoher Reinheit.

Es ist bekannt, dass eine Temperaturerhöhung normalerweise zwar zu einer Erhöhung der Reaktionsgeschwindigkeit führt, aber auch die gleichzeitige Bildung von Nebenprodukten beschleunigt, wie beispielsweise bei der Alkylierung von Phenol mit Diisobuten die unerwünschte Bildung von p-tert.-Butylphenol. Eine Erhöhung des Druckes erhöht die Löslichkeit der flüchtigen Bestandteile, wie Isobuten in der flüssigen Phase. Man musste daher annehmen, dass eine Erhöhung des Druckes ebenfalls die Bildung von p-tert.-Butylphenol begünstigt. Es war daher besonders überraschend, dass man bei gleichzeitiger Erhöhung der Temperatur und des Druckes in Gegenwart von Wasser p-tert.-Octylphenol in hoher Ausbeute erhält bei nur geringem Anfall an p-tert.-Butylphenol. Ohne Zusatz von Wasser erhält man höhere Anteile an Butylphenol (s. Vergleichsbeispiele A bis D).

Im Vergleich zum Verfahren der FR-PS Nr. 2228749 erhält man bei dem erfindungsgemässen Verfahren eine wesentlich höhere Raum-Zeit-Ausbeute an p-Octylphenol. Während man aus dem Beispiel 1 der FR-PS Nr. 2228749 nur eine Raum-Zeit-Ausbeute von 0,65 kg p-Octylphenol/(1-Kontakt, h) errechnen kann, erreicht man bei dem erfindungsgemässen Verfahren Raum-Zeit-Ausbeuten von 8,62 kg/(1-Kontakt, h) (s. Beispiel 4).

Das als unerwünschtes Nebenprodukt anfallende Dioctylphenol setzt man in einer zweiten Stufe in einem weiteren Reaktor mit Phenol in Gegenwart eines sauren Ionenaustauschers unter Zusatz von Wasser bei Temperaturen von 110 bis 140°C und einem Überdruck bis zu 5 bar zum p-tert.-Octylphenol um. Überraschenderweise führt diese Umsetzung in Gegenwart von Wasser praktisch nur zum p-tert.-Octylphenol (s. Beispiele 1 bis 4), während man ohne Wasser als Hauptprodukt p-tert.-Butylphenol erhält (s. Vergleichsbeispiele A bis D).

Das Verfahren kann kontinuierlich und diskontinuierlich durchgeführt werden. Als Katalysatoren setzt man saure Ionenaustauscher ein, insbesondere Harze vom Typ des sulfonierten, mit Divinylbenzol vernetzten Polystyrols. Diese Katalysatoren setzt man in beiden Stufen im allgemeinen in Mengen von 5 bis 8 Gew.-%, bezogen auf das eingesetzte Phenol bzw. die stündlich durchgesetzte Phenolmenge, ein. Unterschiedlich zum Verfahren der FR-PS Nr. 2228749 setzt man diese Katalysatoren trocken ein.

Das Molverhältnis Phenol zu Diisobuten beträgt allgemein bis 8:1, vorzugsweise bis 2:1, das Molverhältnis Phenol zu Dioctylphenol liegt allgemein bei 1:1 bis 8:1, bevorzugt bei 2:1 bis 3:1.

Man arbeitet in beiden Reaktionsstufen bei einem Zusatz von 1 bis 5% Wasser, bezogen auf das Einsatzgemisch Vorzugsweise setzt man 2 bis 3% Wasser zu.

Die Umsetzung erfolgt bei Temperaturen von 110 bis 140, bevorzugt bei 115 bis 130°C bei einem Überdruck bis zu 5, vorzugsweise von 1,5 bis 2,5 bar. Die Reaktionszeit bzw. die Verweilzeit liegt im allgemeinen bei 3 bis 60, bevorzugt bei 8 bis 12 min.

Das vereinfachte Fliessbild zeigt die kontinuierliche Durchführung des Verfahrens: Die beiden Reaktoren R1 und R2 sind Festbettreaktoren und mit einem sauren Ionenaustauscher gefüllt. Aus Vorratstanks werden Phenol, Diisobuten und Wasser in den Reaktor 1 gegeben. Das austretende Reaktionsgemisch wird über einen Rohaustragsbehälter B1 in den Destillationsteil gefahren, wo in der ersten Kolonne K1 zunächst Olefin und Wasser abgetrennt und vor den ersten Reaktor zurückgefahren werden. In der zweiten Kolonne K2 wird Phenol mit den gebildeten Butylphenolen über Kopf genommen und der Aufarbeitung unterworfen. Das Sumpfprodukt wird in einer weiteren Kolonne K3 in einen sog. Vorlauf, der hauptsächlich niedrig siedende Octylphenolisomere enthält, und p-tert.-Octylphenol mit den gebildeten Polyalkylphenolen zerlegt. Eine weitere Kolonne K4 liefert am Kopf das Reinprodukt; die am Kolonnensumpf abgezogene Fraktion wird in einem Dünnschichtverdampfer aufgearbeitet. Das als Destillat anfallende Dioctylphenol fährt man vor den zweiten Reaktor R2 zur Umalkylierung. In diesen Umalkylierungsreaktor werden zusätzlich Phenol und Wasser geführt. Das Reaktionsgemisch aus R2 führt man in den Rohaustragsbehälter B1 und arbeitet es anschliessend wie beschrieben in der Destillationsanlage auf.

Ein weiterer Vorteil des Verfahrens ist es, dass man ein sehr reines p-tert.-Octylphenol mit einem Erstarrungspunkt von ca. 80°C erhält. Das in hoher Ausbeute erhaltene reine p-tert.-Octylphenol verwendet man zur Herstellung von Emulgatoren, Waschrohstoffen und Phenolplasten.

*Beispiele 1 bis 3:*

a) *Erste Stufe*

Die Umsetzung erfolgt diskontinuierlich in einem Rührkolben, der mit Kontaktthermometer und Rückflusskühler ausgerüstet ist, unter den in der Tabelle 1 angegebenen Bedingungen. Man legt 90 g eines sulfonierten Polystyrol-Ionenaustauscherharzes in der H-Form, das zu 8 Gew.-% mit Divinylbenzol vernetzt ist und eine Aktivität von 4,6 mVal/g Katalysator hat, vor und gibt 282 g Phenol und 168 g Diisobuten (Molverhältnis Phenol/Diisobuten 2:1) und 9 g Wasser (2 Gew.-%, bezogen auf das Gemisch Phenol/Diisobuten) zu. Nach einer Reaktionszeit von 15 min wird der Rohaustrag durch Filtration vom Kontakt getrennt und in bekannter Weise ohne jede Vorbehandlung destillativ aufgearbeitet. Man erhält ein p-Octylphenol mit einem sehr hohen Reinheitsgrad (Erstarrungspunkt 80,2°C).

*(Tabelle auf der nächsten Seite)*

b) *Zweite Stufe*

Die Umsetzung in der zweiten Stufe erfolgt ebenfalls diskontinuierlich in einem Rührkolben, der mit Kontaktthermometer und Rückflusskühler

*Tabelle 1*

| Beispiel | Temp. (°C) | Druck (bar) | Umsatz (%) | Zusammensetzung (Gew.-%, phenolfrei gerechnet) | | | Rest |
|---|---|---|---|---|---|---|---|
| | | | | Butylphenol | Octylphenol | Dioctylphenol | |
| 1 a | 110 | 1,5 | 79,1 | 4,1 | 85,8 | 5,3 | Zwischenläufe |
| 2 a | 110 | 2,5 | 84,0 | 3,9 | 86,8 | 4,5 | |
| 3 a | 130 | 2,3 | 88,5 | 8,7 | 86,5 | 4,75 | |

ausgerüstet ist, unter den in der Tabelle 2 angegebenen Bedingungen. Man legt 75 g des in der ersten Stufe eingesetzten stark sauren Ionenaustauschers vor und gibt 446 g Phenol und 377 g des in den Beispielen 1 a bis 3 a erhaltenen Dioctylphenols (Molverhältnis Dioctylphenol/Phenol 1:4) und 16 g Wasser (2,0 Gew.-% Wasser, bezogen auf das Gemisch Phenol/Dioctylphenol) zu. Nach einer Reaktionszeit von 60 min arbeitet man ohne jede Vorbehandlung in bekannter Weise auf.

*Tabelle 2*

| Beispiel | Temp. (°C) | Druck (bar) | Umsatz (%) | Zusammensetzung (Gew.-%, phenolfrei gerechnet) | | |
|---|---|---|---|---|---|---|
| | | | | Butylphenol | Octylphenol | Dioctylphenol |
| 1 b | 110 | 1,5 | 99,2 | 0,99 | 67,6 | 30,0 |
| 2 b | 110 | 2,5 | 99,4 | 2,09 | 64,1 | 32,8 |
| 3 b | 130 | 2,3 | 64,0 | 3,7 | 55,8 | 34,4 |

*Beispiel 4:*

An den Reaktor R1 (s. Zeichnung) legt man 70 kg ($\cong$ 87 l) des in den Beispielen 1 bis 3 eingesetzten stark sauren Ionenaustauschers vor und gibt

   760 kg/h Phenol
   458 kg/h Diisobuten
   23 kg/h Wasser zu

Die Temperatur des Reaktionsgemisches vor Eintritt in den Reaktor wird so geregelt, dass die Austrittstemperatur 130°C beträgt; den Druck hält man auf 2,3 bar. Das Rohalkylat arbeitet man in den Kolonnen K1 bis K4 in bekannter Weise auf und erhält 750 kg p-Octylphenol/h. Das im anschliessenden Dünnschichtverdampfer (DSV) anfallende Dioctylphenol fährt man vor den zweiten Reaktor R2, in dem man das Dioctylphenol mit Phenol und Wasser nach den Angaben der Beispiele 1 b bis 3 b bei 130°C und 2,3 bar umalkyliert.

*Vergleichsbeispiele A bis D:*

a) *Erste Stufe*

Nach den Angaben der Beispiele 1 bis 3 setzt man die dort eingesetzten Mengen an Phenol und Diisobuten in Gegenwart des dort angegebenen stark sauren Ionenaustauschers und gegebenenfalls in Gegenwart von Wasser um. Die Aufarbeitung erfolgt den Angaben der Beispiele 1 bis 3 entsprechend. Die Ergebnisse sind in den Tabellen 3 und 4 zusammengestellt. Man erhält bei tieferen Temperaturen unter Atmosphärendruck erheblich niedrigere Umsätze. Ohne Zusatz von Wasser ist der Anteil an unerwünschtem Butylphenol wesentlich höher.

*Tabelle 3*

| Vergleichsbeispiel | Wasser (Gew.-%) | Temp. (°C) | Druck (bar) | Umsatz (%) | Zusammensetzung (Gew.-%, phenolfrei gerechnet) | | |
|---|---|---|---|---|---|---|---|
| | | | | | Butylphenol | Octylphenol | Dioctylphenol |
| $A_1$ | 2 | 90 | 1 | 37,2 | 8,4 | 82,9 | 6,0 |
| $B_1$ | 0 | 90 | 1 | 34,4 | 14,1 | 81,5 | 2,2 |
| $C_1$ | 0 | 110 | 1,4 | 80,5 | 10,4 | 84,4 | 2,3 |
| $D_1$ | 0 | 130 | 2,1 | 94,1 | 28,6 | 68,8 | 2,5 |

b) *Zweite Stufe*

Die Umsetzung von Dioctylphenol mit Phenol erfolgt ebenfalls nach den Angaben der Beispiele 1 bis 3, jedoch ohne Zusatz von Wasser.

*Tabelle 4*

| Ver-gleichs-beispiel | Temp. (°C) | Druck (bar) | Umsatz (%) | Zusammensetzung (Gew.-%, phenolfrei gerechnet) | | |
|---|---|---|---|---|---|---|
| | | | | Butylphenol | Octylphenol | Dioctylphenol |
| D$_2$ | 130 | 2,3 | 99,7 | 74,7 | 18,9 | 0,4 |

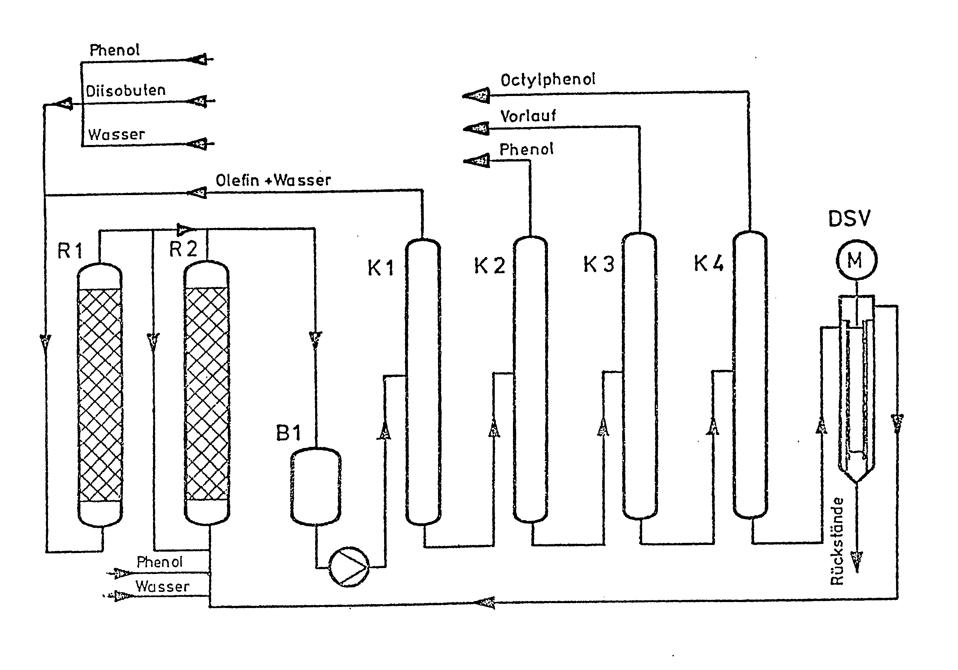

## Patentansprüche

1. Verfahren zur Herstellung von p-tert.-Octylphenol durch Umsetzen von Phenol mit Diisobuten in Gegenwart eines sauren Ionentauschers unter Zusatz von Wasser, dadurch gekennzeichnet, dass man den sauren Ionenaustauscher trocken einsetzt, die Reaktion in einer ersten Stufe unter Zusetzen von 1 bis 5% Wasser, bezogen auf das Einsatzgemisch, in einem Festbettreaktor bei Temperaturen von 110 bis 140°C unter Überdruck bis zu 5 bar durchführt und man das als Nebenprodukt erhaltene Dioctylphenol in einer zweiten Stufe in einem weiteren Festbettreaktor mit Phenol in Gegenwart eines sauren Ionenaustauschers unter Zusatz von 1 bis 5% Wasser, bezogen auf das Einsatzgemisch, bei Temperaturen von 110 bis 140°C und einem Überdruck bis zu 5 bar zum p-tert.-Octylphenol umsetzt.

2. Verfahren nach Anspruch 1, dadurch gekennzeichnet, dass man bei einer Temperatur von 115 bis 130°C arbeitet.

3. Verfahren nach den Ansprüchen 1 und 2, dadurch gekennzeichnet, dass man die Reaktion bei einem Druck von 1,5 bis 2,5 bar in den Reaktoren durchführt.

## Claims

1. A process for the production of p-tert.-octylphenol by reaction of phenol with diisobutene in the presence of an acidic ion exchanger with the addition of water, characterised in that the acidic ion exchanger is introduced dry, the reaction is carried out in a first stage in a fixed bed reactor at a temperature of 110 to 140°C under a superatmospheric pressure up to 5 bar with the addition of 1 to 5% of water based on the starting mixture, and the dioctylphenol obtained as byproduct is reacted with phenol to form p-tert.-octylphenol in a second stage in a further fixed bed reactor at a temperature of 110 to 140°C and a superatmospheric pressure of up to 5 bar in the presence of an acidic ion exchanger with the addition of 1 to 5% of water based on the starting mixture.

2. A process according to Claim 1, characterised in that it is operated at a temperature of 115 to 130°C.

3. A process according to Claims 1 and 2, characterised in that the reaction is carried out at a pressure of 1.5 to 2.5 bar in the reactors.

## Revendications

1. Procédé de préparation de p-tert.-octylphénol par réaction du phénol sur le diisobutène en présence d'un échangeur d'ions acide avec addition d'eau, caractérisé en ce que l'on utilise à sec l'échangeur d'ions acide, que l'on conduit la réaction à un premier stade avec addition de 1 à 5% d'eau relativement au mélange initial, dans un réacteur à couche fixe, à des températures de 110 à 140°C, sous une surpression allant jusqu'à 5 bar et que, à un deuxième stade, on fait réagir le dioctylphénol obtenu comme sous-produit, dans un autre réacteur à couche fixe, sur le phénol en présence d'un échangeur d'ions acide avec addition de 1 à 5% d'eau relativement au mélange initial, à des températures de 110 à 140°C et à une surpression allant jusqu'à 5 bar, pour obtenir le p-tert.-octylphénol.

2. Procédé selon la revendication 1, caractérisé en ce que l'on opère à une température de 115 à 130°C.

3. Procédé selon les revendications 1 et 2, caractérisé en ce que l'on conduit la réaction à une pression de 1,5 à 2,5 bar dans les réacteurs.

Phenol

Diisobuten

Wasser

Octylphenol

Vorlauf

Phenol

Olefin + Wasser

R 1

R 2

K 1

K 2

K 3

K 4

DSV

M

B 1

Phenol

Wasser

Rückstände

0 083 700